(19) Europäisches Patentamt
European Patent Office
Office européen des brevets

(11) EP 1 251 858 B1

(12) EUROPEAN PATENT SPECIFICATION

(45) Date of publication and mention of the grant of the patent:
**08.06.2005 Bulletin 2005/23**

(21) Application number: **01900598.2**

(22) Date of filing: **11.01.2001**

(51) Int Cl.$^7$: **A61K 31/7004**, A61K 9/107, A61K 35/78, A61P 35/00

(86) International application number:
**PCT/IB2001/000087**

(87) International publication number:
**WO 2001/052826 (26.07.2001 Gazette 2001/30)**

# (54) PODOPHYLLOTOXIN COMPOSITIONS

PODOPHYLLOTOXIN-ZUSAMMENSETZUNGEN

COMPOSITIONS DE PODOPHYLLOTOXINE

(84) Designated Contracting States:
**AT BE CH CY DE DK ES FI FR GB GR IE IT LI LU MC NL PT SE TR**
Designated Extension States:
**AL LT LV MK RO SI**

(30) Priority: **20.01.2000 US 488298**

(43) Date of publication of application:
**30.10.2002 Bulletin 2002/44**

(73) Proprietor: **SUPRATEK PHARMA INC.**
**Montreal, Quebec H2Y 1M9 (CA)**

(72) Inventors:
- **LUTZ, Olivier**
  **Laval, Quebec H7V 1J8 (CA)**
- **KLINSKI, Evgueni**
  **Laval, Quebec H7V 1J8 (CA)**
- **LI, Shengmin**
  **St-Laurent, Quebec H4N 2H4 (CA)**
- **ALAKHOV, Valery**
  **Montreal, Quebec H9X 3V4 (CA)**

(74) Representative: **Keen, Celia Mary et al**
**J.A. Kemp & Co.**
**14 South Square**
**Gray's Inn**
**London WC1R 5JJ (GB)**

(56) References cited:
**WO-A-97/10849**
**WO-A-99/45918**
**US-A- 5 534 499**
**US-A- 5 891 469**
**US-A- 5 891 845**

- **CHEMICAL ABSTRACTS, vol. 124, no. 26, 24 June 1996 (1996-06-24) Columbus, Ohio, US; abstract no. 352450a, K. SON ET AL.: "Physical stability of teniposide in bile salt-egg phosphatidylcholine mixed micelles and liposomes" page 655; XP002180005 & PDA J. PHARM. SCI. TECHNOL., vol. 50, no. 2, 1996, pages 89-93,**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 251 858 B1

## Description

FIELD OF THE INVENTION

The invention relates to compositions of podophyllotoxins, analogues thereof or derivatives thereof with improved therapeutic properties. The present invention also relates to the use of such podophyllotoxin compositions in the manufacture of anti-cancer agents.

BACKGROUND OF THE INVENTION

PODOPHYLLOTOXINS

In the field of natural products, podophyllotoxins represent an important chemical class of therapeutic agents. They are naturally-occurring compounds, found in plants, particularly in the genus *Podophyllum.* In 1844, in the first report on extraction of Podophyllum, the resin obtained from alcohol extraction was called podophyllin. In the 19th century, the resin podophyllin was recommended for treating diseases of the liver and kidneys and for scrofula, syphilis, gonorrhea and coughs. In 1942, Chaplains cured the venereal wart *Condyloma accuminata* with application of podophyllin in oil. This led to studies of the action of podophyllin on tumor tissues and to the chemical examination of podophyllin. A number of laboratories, particularly the National Cancer Institute, explored the activity and chemistry of many constituents of podophyllin (see *Hartwell and Schrecker, Fortschr. Chem. Org. Naturst.*, 1958, 15 : 83). Podophyllotoxin was isolated from podophyllin in 1880. It belongs to the general structure of an aryltetralin lignan lactone.

Podophyllotoxins have various biological activities. They have attracted considerable interest as human cancer chemotherapeutic agents. *Podophyllum* and podophyllin has been used as a folk medicine for the treatment of cancer for over 1000 years. Severe toxic effects have restricted the use of podophyllotoxin as an anticancer agent. However, podophyllotoxin treatment continues to be the best for treating genital warts (see Benther and Von Krogh, *Seminars in Dermatology,* 1990, 9 : 148).

New podophyllotoxin derivatives have been developed to produce less toxic side effects. Two drugs have emerged, the epipodophyllotoxins, etoposide (VP-16) and teniposide (VM-26), which have wide spectrum antitumor activity. These two compounds are currently used for the treatment of a variety of cancers. Etoposide exhibits significant activity against oat carcinoma of the lung and ovarian cancer. Further, Etoposide is one of the most active single agents against small cell bronchus carcinoma, testicular carcinoma, choriocarcinoma and neuroblastoma (see Schmoll *et al*., *Klinische Wochenschrift*, 1981, 59: 1177). It is also effective against myelocytic and monocytic malignancies in children. As part of a combination therapy, etoposide had become almost a standard in therapies for small cell lung cancer and testicular cancer. Teniposide is effective against acute lymphocyte leukemia, myelogenous leukemia, Hodgkin's diseases, histocytic lymphoma, Wilm's tumor, Ewing's sarcoma, and sarcoccygeal sarcoma (see Bleyer *et al*., *Cancer Treatment Reports,* 1979, 63: 977). Both compounds are potent in the treatment of acute leukemia.

In addition of their antitumor activity, podophyllotoxins show significant antiviral activity. They have been reported to be active agents particularly against herpes simplex type-1 virus (HSV-1) (see Hammonds *et al*., *J. Med. Microb*., 1996, 45: 167), against murine cytomegalovirus (CMV), and sindbis virus (see Mac Rae *et al*., *Planta Medica*, 1989, 55: 531).

Podophyllotoxin and its isomers were tested for other activities like insecticidal activity, phytogrowth inhibitory activity and ichthytoxic activity.

Regarding the mechanism of action on cell growth, most podophyllotoxin drugs, including VP-16 (commercial formulation of etoposide; VEPESID®; Bristol-Myers Squibb) and VM-26 (commercial formulation of teniposide; VUMON®; Bristol-Myers Squibb) are DNA topoisomerase II inhibitors. The compounds act by causing single and double strand breaks in DNA (see Loike and Horwitz, *Biochemistry*, 1976, 15: 5443-48). Podophyllotoxin, their parent compound, acts by an entirely different kind of mechanism. It acts as a potent inhibitor of microtubule assembly by binding to the tubulin dimers.

Multiple drug resistance is often found in many types of human tumors that have relapsed after an initial positive response to chemotherapy (see Goldstein *et al*., *Cancer Treat. Res.,* 1991, 57: 101; Goldstein *et al*., *Crit. Rev. Oncol. Hematol*., 1992, 12: 243). It may be attributed to alterations in the flux of drugs across the cell plasma membrane. Resistant cells accumulate less drug than susceptible cells and have the ability to pump the drug out. Resistance mechanisms involve the over expression of a membrane pump, glycoprotein P (P-gp). By reducing the cellular net accumulation of the drug, P-gp contributes to tumor resistance to chemotherapy. In addition to the reduced intracellular concentration of the drugs, other resistance mechanisms have been reported for etoposide such as a qualitative change or reduction in topoisomerase II activity and the deactivation of the drug (see Lock and Hill, *Int. J. Cancer,* 1988, 42: 373-81). It has also been shown *in vivo* that long treatment with VP-16 induced crossed resistance to other anticancer drugs, such as anthracyclines and vindesine, and partial crossed resistance with daunorubicin. Furthermore, there is

evidence that delivery vehicles effective in enhancing the activity of a chemotherapeutic agent may also have the ability of reversing resistance to such an agent. For example, pluronic block copolymers were shown to sensitize multidrug resistant cancer cells (Alakhov *et al*., 1996, *Bioconjugate Chem*. 7:209).

Podophyllotoxins exhibit some toxic effects. The dose-limiting toxicity of epipodophyllotoxins is myelosuppression, mainly leukopenia (see Wolf *et al*., *J. Clin. Oncol*., 1987, 5: 1880-89). Other less severe toxic effects include gastrointestinal effects as occasional diarrhea, stomatitis, mucositis, and alopecia. There is no report on neurotoxicity of etoposide, and hypersensitivity reactions to etoposide have rarely been reported. Teniposide toxicities are identical to those of etoposide although allergic reactions are seen more frequently.

Podophyllotoxins exhibit poor biodistribution, for example, very low or unquantifiable concentrations of etoposide or teniposide are found in cerebrospinal fluid after intravenous administration in children and adults, whereas there is some penetration of these drugs in brain tumors (see Stewart *et al*., *J. Neuro-oncology*, 1984, 2(2): 133-39; Stewart *et al*., *J. Neuro-oncology*, 1984, 2(4): 315-24). Low concentrations of both compounds have been reported in pleural and ascitic fluids. Etoposide and its hydroxy metabolite have been detected in liver, spleen, renal tissues, lung, myometrium, subcutaneous tissues and saliva. Furthermore, etoposide and teniposide have been shown to penetrate into liver, kidney, spleen, brain tissue, heart and intestine of mice.

Due to a low aqueous solubility, administration of podophyllotoxins is associated with clinical problems. Etoposide, which has a limited chemical and physical stability in water, requires formulations with complex mixtures of alcohols and surfactants for intravenous administration (see, for example, Beijnen *et al*., *J. Parenteral Science & Technology,* 1991, 45:108-12). Another way to overcome the solubility problems is to modify the molecule. More recently, the 4'-phosphate ester of etoposide has been developed as a prodrug of the parent compound for intravenous use (ETOPOPHOS®, Bristol-Myers Squibb Co, Princeton, NJ, USA, see Witterland *et al*., *Pharmacy World & Science,* 1996, 18(5): 163-70). This derivative exhibits a better aqueous solubility but must be rapidly converted *in vivo* to etoposide by alkaline phosphatases in the blood to exert its cytotoxic activity.

The more convenient oral administration of etoposide has been widely investigated. There is no pharmacological difference between oral and intravenous administration of etoposide with respect to drug mechanism of action, half-life, mode of drug elimination or type of toxicity. Several oral formulations have been evaluated. However, all these formulations yielded poor oral bioavailabilities with high intra- and interpatient variabilities in the rate of etoposide absorption. One reason is that when etoposide is diluted with aqueous solvents such as gastric and intestinal secretions, its solubility is inevitably compromised. Bioavailability of oral etoposide was reported to range from 40 to 75% depending on the drug dose (see Hande *et al*., *J. Clin. Oncol*., 1993, 11: 374 -77).

Thus, it would be beneficial to have new formulations of podophyllotoxins to overcome the problems of its low solubility and its irregular and unpredictable precipitation from an aqueous environment; and to improve its antitumor activity.

SURFACTANTS

Surfactants, such as d-α-tocopheryl polyetherylene glycol 1000 succinate (TPGS) and the like, were used in the past as a drug delivery vehicles either as an emulsifier or a solubilizer, and TPGS is considered to be an absorption enhancer when administered with some lipophilic drugs. However, the compounds of the present invention differ dramatically from those known previously.

U.S. Patent No. 5,798,333 issued to Sherman describes water-soluble compositions of cyclosporins dissolved in tocophersolan and a hydrophilic organic solvent. Unlike that previously described in Sherman, the compositions of the present invention dispense with the use of a co-solubilizer or a hydrophilic organic solvent. Moreover, the present method of preparation does not consist of melting TPGS above 36°C and dissolving the drug into the melted TPGS. In the present invention, both TPGS and a drug are dissolved in an organic solvent that is distilled off under vacuum.

US Pat. No. 4,578,391 issued to Kawata *et al*. Describes oily compositions of an anti-tumor drug comprising certain sparingly oil-soluble or water-soluble anti-tumor drugs, an effective amount of a fat or oil, and an effective amount of certain solubilizing adjuvants. Unlike that previously described in Kawata, the present invention dispenses with the use of a fat or oil in the composition. Also, the present invention provides a composition in a micellar solution form and not in an oil-in-water emulsion form.

US Pat. No. 5,886,030 issued to Maniar and Manoj describes the use of Vitamin E tocopheryl derivatives in ophthalmic compositions, which increases the solubility of certain poorly soluble ophthalmic agents. Maniar describes only therapeutic selected from the group of non-steroidal anti-inflammatory agents and steroidal anti-inflammatory agents. Unlike previously described in Maniar, the present invention includes drugs belonging preferably to the group of podophyllotoxin derivatives. In addition, in the present invention, the drug is not dissolved in a TPGS water solution. Instead, both TPGS and a drug are dissolved in an organic solvent that is distilled off under vacuum, and the drug/ TPGS solid solution obtained is then dispersed with phosphate buffer saline (PBS) or another aqueous medium.

US Pat. No. 5, 891,469 issued to Amselem describes solid compositions comprising a lipophilic substance

and tocopherol polyetheylene glycol succinate (TPGS) and an adjuvant. Amselem also describes methods of preparing these solid compositions by comelting the TPGS and lipophilic substance, adding the adjuvant, and drying the mixture. Unlike that previously described in Amselem, the composition of the present invention comprises two ingredients, a derivative of podophyllotoxin or another lipophilic drug, and TPGS or its derivative. Preferably, the present invention uses the micellar microphase formed by TPGS at the concentration range of 0.02 wt % up to 10 wt % that provides highly stable spontaneously formed particles of a small size. Moreover the formulation of the present invention is prepared without co-melting TPGS and a lipophilic drug but by using an organic solvent evaporation technique.

US Patent No. 5,891,845 issued to Myers describes compositions of vitamin E TPGS and one or more lipophilic drugs in a solid solution. Unlike that previously described in Myers, the present invention does not relate to pharmaceutical drug compositions and delivery devices that form liquid crystal structures with TPGS. The compositions of the present invention provide aqueous micellar solutions of drug/TPGS.

Citation of a reference hereinabove shall not be construed as an admission that such a reference is prior art to the present invention.

## SUMMARY OF THE INVENTION

The present invention provides a composition comprising an aqueous dispersion of micelles having an average diameter of less than 300 nm, said micelles comprising (a) a podophyllotoxin selected from etoposide and teniposide and analogues and derivatives thereof; and (b) tocoferol covalently linked to a water-soluble polymer.

In one embodiment the composition of the invention as defined above further comprises a targeting molecule or moiety.

The invention also provides a composition of the invention as defined above for the treatment of the human or animal body by therapy.

The present invention also provides for a process of delivering to a cell, *ex vivo*, a podophyllotoxin selected from etoposide and teniposide and analogues and derivatives thereof, which process comprises administering to the cell a composition of the invention as defined above. Further, the present invention provides the use, in the manufacture of a medicament for use in the treatment of cancer, of an aqueous dispersion of micelles having an average diameter of less than 300 nm, said micelles comprising:

(a) a podophyllotoxin selected from etoposide and teniposide and analogues and derivatives thereof; and
(b) tocoferol covalently linked to a water-soluble polymer.

In a preferred embodiment of the invention the tocoferol covalently linked to a water soluble polymer is d-$\alpha$-tocopheryl polyethylene glycol 1000 succinate (TPGS) or a derivative thereof. Preferably, the TPGS is present at a concentration of from 0.02 wt % to 20 wt %. More preferably, the TPGS is present at a concentration of from 0.02 wt % to 10 wt %. Typically, the TPGS is present at a concentration of from 4 wt % to 10 wt %.

In another preferred embodiment of the invention, the micelles of the composition have an average diameter of less than 100 nm. More preferably, the average diameter is less than 50 nm. Typically, the average diameter is from 3 nm to 25 nm.

The invention is based in part, on a number of unanticipated surprising discoveries. One is the discovery that the ability of TPGS to solubilize a significant amount (for example, 10 mg/ml of podophyllotoxin) by forming a stable dispersion, is essential in view of the current invention. Other ionic and non-ionic surfactants, for example, PLURONIC P85, PLURONIC 127, PLURONIC 123, PLURONIC 68, PLURONIC 108, 88, 61, polyethyleneimine-polyoxyethylene do not solubilize podophyllotoxin. Furthermore, a podophyllotoxin formulation for administration in the body needs to be chemically and thermodynamically stable. For example, 80% solutions of TWEEN-80 in alcohols are capable of solubilizing podophyllotoxin at a concentration of 24 mg/ml. Before administration into the body this solution must be diluted with an aqueous solution such as an isotonic solution. However, after such a dilution the podophyllotoxin precipitates within several hours. This severely limits therapeutic use of the existing podophyllotoxin formulations. The podophyllotoxin formulation of the current invention is stable at room temperature for at least 3 days. During storage of the present formulation, no chemical decomposition of the podophyllotoxin is observed. These findings together indicate that the formulations of the current invention are novel.

The invention has utility in providing compositions of podophyllotoxins in micelle form. Such compositions are useful for example, for administration to an animal or patient for treatment of diseases, inhibiting cancer, or delivering podophyllotoxins to a cell.

## DEFINITIONS

The terms or phrases listed below shall have the following meaning :

Biological agent: An agent that is useful for treating, diagnosing or imaging or that can act on a cell, organ or organism, including but not limited to drugs (pharmaceuticals) to create a change in the functioning of the cell, organ or organism.

Chemotherapeutic agent: A biological agent that inhibits the growth or decreases the survival of neoplastic or pathogenic microbial cells or inhibits the propagation (which includes without limitation replication, viral assembly or cellular infection) of a virus.

Hydrophobe percentage: The percentage of the molecular weight of a block copolymer that is made up of B-type blocks.

Hydrophobe weight: The molecular weight contribution of the B-type blocks of a block copolymer. $IC_{50}$: The concentration at which 50% cytotoxicity is obtained. Cytotoxicity can be measured by the method of Alley *et al.*, *Cancer Res.,* 48: 589-601 (1988) or Scudiero *et al.*, *Cancer Res.,* 48: 4827 (1988). In particular, it can be measured based on the drug concentration at which a 50% reduction in the activity of mitochondrial enzymes is observed.

$IC_{95}$: The concentration at which 95% cytotoxicity is obtained. Cytotoxicity can be measured by the method of Alley *et al.*, above, or Scudiero *et al.*, above. Specifically, it can be measured based upon the drug concentration at which a 95% reduction in the activity of mitochondrial enzymes is observed.

Lipophilic moiety: A lipophilic substituent that is joined to a targeting moiety and that partitions into the lipophilic portion of copolymer micelles.

MDR: The phenomenon of simultaneous resistance to unrelated biological agents.

Targeting moiety: A molecular structure that is recognized by a cellular, tissue, viral or substratum component such as a cell surface receptor or acceptor molecule.

Critical micellar concentration (CMC): The minimal concentration at which a surfactant forms micelles.

Hydrophile/lipophile balance (HLB): A quantitative measure of the hydrophilicity and lipophilicity of a surfactant, and therefore of its solubilization properties. A HLB value between 12 and 14.5 is effective for solubilization of membranes and lipids.

Micelles: Micelles are supramolecular complexes of certain amphiphilic molecules that form in aqueous solutions due to microphase separation of the nonpolar portions of the amphiphiles. Micelles form when the concentration of the amphiphile reaches, for a given temperature, a critical micellar concentration ("CMC") that is characteristic of the amphiphile. The micelles have translational and rotational freedom in aqueous environment, and aqueous environments containing the micelles have low viscosity similar to water.

The effective retention of a drug within the micelles of the invention can be quantified in terms of the partitioning coefficient (P) determined using formula:

$$P = (Agent)_m / (Agent)aq$$

where $(Agent)_{aq}$ is the concentration of biological agent in an aqueous environment outside of the micelles and $(Agent)_m$ is the concentration of agent in the micelles. In some cases, P is easily and accurately estimated based on the difference in the fluorescence properties of certain agents when in an aqueous vs. a more hydrophobic environment. Surfactant: Surface active agent that is adsorbed at interface.

## DETAILED DESCRIPTION OF THE INVENTION

The present invention also provides compositions as defined above for treatment of the human or animal body for use in the manufacture of a medicament for use in inhibiting cancer and delivering podophyllotoxins to cells.

Solely for ease of explanation, and not by way of limitation, the description of the invention is divided into the following sections: (1) podophyllotoxins, (2) surfactants, (3) targeting molecules, and (4) methods of use.

PODOPHYLLOTOXINS

The podophyllotoxin is selected from etoposide and teniposide and analogues and derivatives thereof. Podophyllotoxin is of the formula:

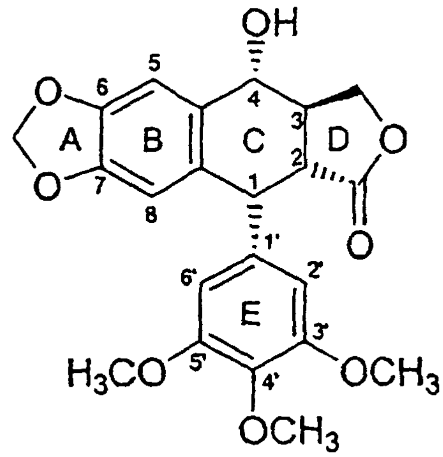

Examples of podophyllotoxins which can be used in this invention are VM-26 (teniposide), VP-16-213, glucoside derivatives of VP-16 and VM-26, acetal and ketal derivatives of 4'-demethylepipodophyllotoxin-β-D-glucoside (DMEPG), the desoxy E-ring analogues of etoposide and 2'-chloro derivatives of etoposide.
Other examples are etoposide and teniposide derivatives with one of the hydroxyl groups in the glycosidic moiety substituted by an alkylamino group.

SURFACTANTS

In this invention the tocoferol covalently linked to a water-soluble polymer that is used includes, but is not limited to TPGS and derivatives of TPGS, which will have modified CMC and HLB, formed by attaching a polymer on the tocopherol succinate portion or by attaching TPGS to the hydroxyl group of polyethylene gylcol (PEG).
In a specific embodiment, the tocoferol covalently linked to a water-soluble polymer to solubilize the podophyllotoxin is d-α-tocopheryl polyethylene glycol 1000 succinate, also known under the name Vitamin E TPGS, or simply TPGS, or listed by the United States Adopted Names as tocophersolan.
In yet another specific embodiment, in the tocoferol covalently linked to a water-soluble polymer, the water-soluble polymer is poly-oxyethylene, poly-oxyethylene-poly-oxypropylene copolymers, polyacrylamides, polyglycerols, polyvinylalcohols, polyvinylpyrrolidones, polyvinylpyridine N-oxides, copolymers of vinylpyridine N-oxide and vinylpyridine, polyoxazolines, polyacroylmorpholines or derivatives thereof.
In a more specific embodiment, the water-soluble polymer is a polypeptide or derivative thereof.
In yet another more specific embodiment, the podophyllotoxin composition comprises a further hydrophobic group other than tocoferol.
In a preferred embodiment, the tocoferol covalently linked to a water-soluble polymer is of the formula:

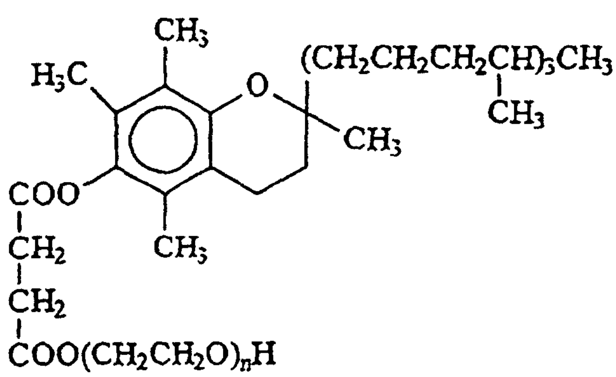

TPGS is a water-soluble derivative of the natural source of vitamin E. It is structurally an amphiphile and it is accepted that the polyethylene glycol portion serves as the hydrophilic part of the molecule while the tocopherol succinate portion serves as the lipophilic part.

Initially, Vitamin E TPGS was used for reversion or prevention of vitamin E deficiency during chronic cholestatis. Additionally, Vitamin E TPGS solubilizes and improves intestinal absorption of other fat-soluble vitamins and medications. It has been clinically demonstrated to enhance the bioavailability of the highly lipophilic drug cyclosporin (see Sokol *et al.*, *The Lancet*, 1991, 338: 212-15).

TPGS has the physical properties to exhibit both a high hydrophile/lipophile balance (HLB) of approximately 13 and a low critical micelle concentration (CMC) of approximately 0.02 wt % at a temperature of 37 °C. Its melting point is approximately 38 °C (100 °F). TPGS exhibits a very high thermal stability under normal processing temperatures used for pharmaceutical applications since its thermal degradation occurs at 199 °C. This characteristic makes it stable under heat sterilization conditions. It forms low viscosity solutions with water until a concentration up to 20 wt %, beyond which liquid crystalline phases may form.

The polymers which can extend the hydrophilic part of the surfactant can include, but are not limited to, other PEG molecules preferably having a molecular weight between 300 and 20000. The hydrophilic portion can also can be extended by polymers and copolymers by using, for example, but not limited to, a diamine linker.

The polymers used in the TPGS derivatives are water-soluble polymers. The preferred nonionic polymer is a homopolymer or copolymer of at least one of the monomers selected from the group consisting of acrylamide , glycerol, vinylalcohol, vinylpyrrolidone, vinylpyridine, vinylpyridine N-oxide, oxazoline, or a acroylmorpholine, and derivatives thereof. This includes, for example, polyacrylamides, polyglycerols, polyvinylalcohols, polyvinylpyrrolidones, polyvinylpyridine N-oxides, copolymers of vinylpyridine N-oxide and vinylpyridine, polyoxazolines, polyacroylmorpholines or derivatives thereof Nonionic segments comprising products of polymerization of vinyl monomers are also preferred, including but not limited to, the following nonionic polymer segments and derivatives thereof having the formulas:

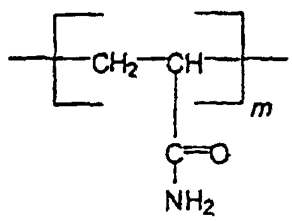

,

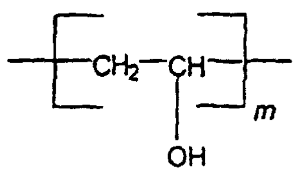

,

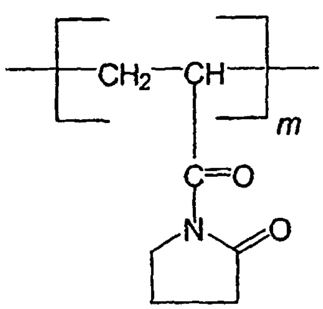

,

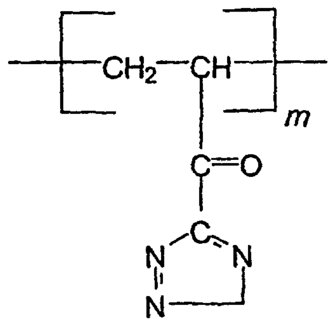

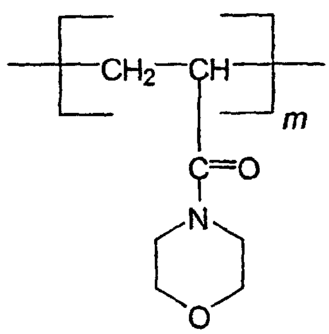

,

or

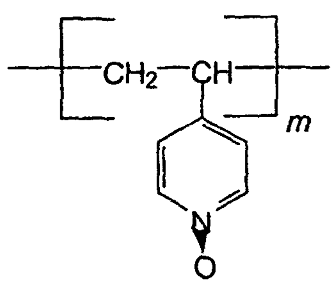

,

in which *m* has a value of from 3 to about 10,000.

In another specific embodiment of the invention, the tocoferol covalently linked to a water-soluble polymer is a TPGS derivative. More particularly, the TPGS is attached covalently to nonionic polymers to the tocopherol succinate portion in place of the polyethylene glycol portion.

These nonionic polymers include, but are not limited to, the group of water-soluble polymers which are nontoxic and non-immunogenic. The preferred nonionic polymer segment, which is a water-soluble nonionic polymer segment, is a homo- or copolymer of at least one of the monomers selected from the group consisting of acrylamide, glycerol, vinylalcohol, vinylpyrrolidone, vinylpyridine, vinylpyridine N-oxide, oxazoline, or a acroylmocgholine, and derivatives thereof. Nonionic segments comprising products of polymerization of vinyl monomers are also preferred.

In another specific embodiment of the invention, the nonionic polymers attached to the tocopherol succinate portion of TPGS also include polyether block copolymers. The polyether block copolymer is selected from the group consisting of polymers having the formulas:

A-B-A', A-B, B-A-B', or $L(R^1)(R^2)(R^3)(R^4)$

(I) (II) (III) (IV)

wherein A and A' are A-type linear polymeric segments, B and B' are B-type linear polymeric segments, and $R^1$, $R^2$, $R^3$ and $R^4$ are either block copolymers of formulas (I), (II) or (III) or hydrogen and *L* is a linking group, with the proviso that no more than two of $R^1$, $R^2$, $R^3$ or $R^4$ are hydrogen.

The hydropbobe weight of the copolymer is at least about 900, more preferably, at least about 1700, yet more preferably at least about 2000, still more preferably at least about 2300. The hydrophobe weight is at least about 2000 and the hydrophobe percentage is at least about 20%, preferably 35%; or the hydrophobe weight is at least about 2300 and the hydrophobe percentage is at least about 20%, preferably 35%.

The copolymer or copolymers of the composition have a critical micellar concentration ('CMC') of no more than about 0.5% wt/vol at 37°C in an isotonic aqueous solution, preferably, no more than about 0.05% wt/vol., more preferably, no more than about 0.01% wt/vol., yet more preferably, no more than about 0.003% wt/vol.

Preferably, the copolymers of the composition conform to Formula (V), which is set forth in the text below. Particularly preferred among these copolymers are those having hydrophobe weights between about 1500 and about 2000, preferably between about 1710 and about 1780, and hydrophobe percentages between about 85% and about 95%, preferably between about 88% and about 92%. Also particularly preferred among these copolymers are those having hydrophobe weights between about 3000 and about 3500, preferably between about 3200 and about 3300, and hydrophobe percentages between about 15% and about 25%, preferably between about 18% and about 22%. Additionally, particularly preferred among these polymers are that having hydrophobe weights between about 3500 and about 4000, preferably between about 3700 and about 3800, and hydrophobe percentages between about 25% and about 35%, preferably between about 28% and about 32%.

In a more specific embodiment of the invention, the preferred block copolymers are of the formula:

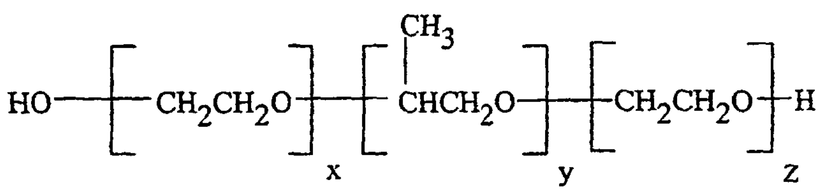

;

(V)

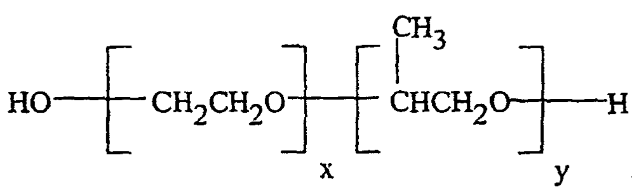

;

(VI)

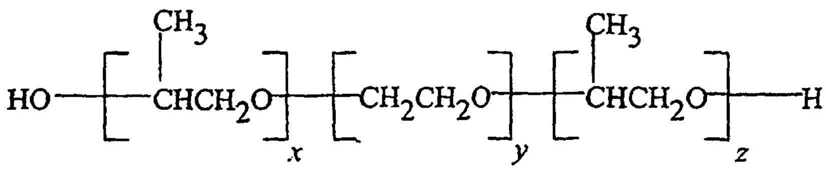

;

(VII)

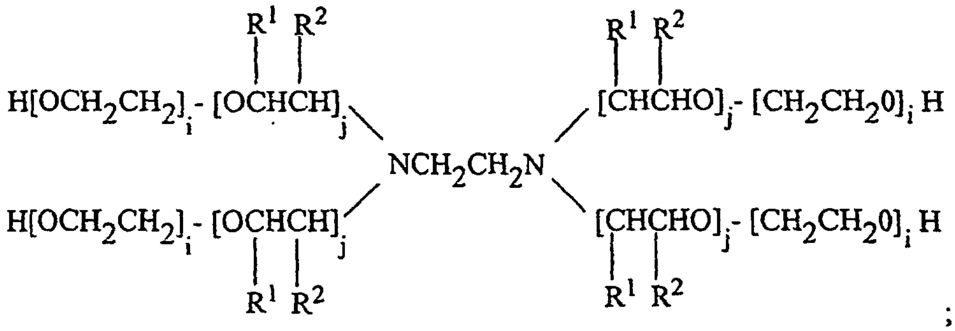

;

(VIII)

or

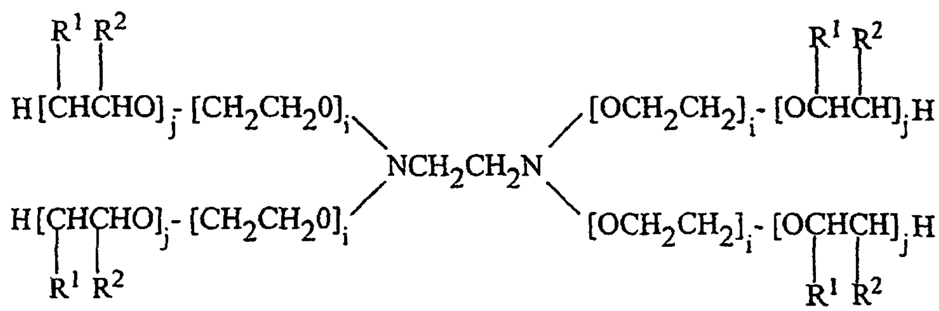

(IX)

in which *x, y, z, i,* and *j* have values from about 2 to about 400, and wherein for each $R^1$, $R^2$ pair, one is hydrogen and the other is a methyl group.

The block copolymer is also of the formula:

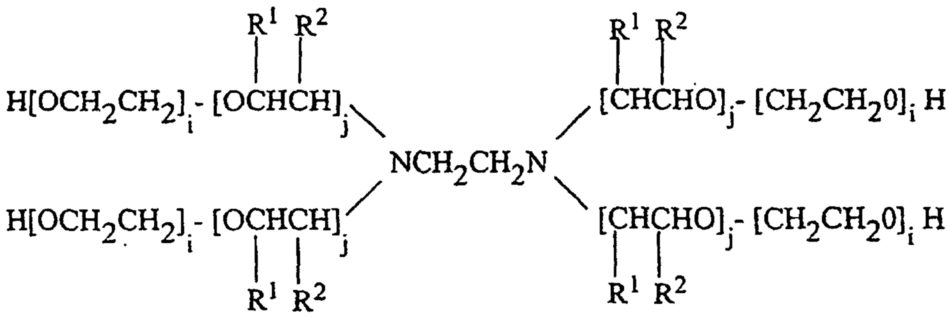

;

(VIII)

or

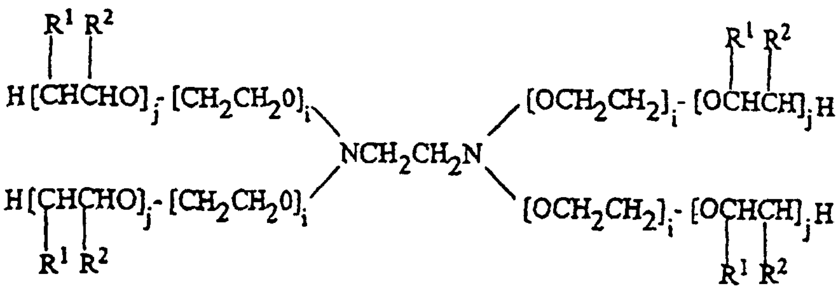

(IX)

wherein for each $R^1$, $R^2$ pair, one is hydrogen and the other is a methyl group. Preferred are those block copolymers wherein the ethylene(oxide) content of said block copolymer is less than 50%.

Further, an embodiment of the present invention includes block copolymer derivatives of TPGS of formulas (V)-(IX) in which *x, y, z, i*, and *j* have values from about 2 to about 400, preferably from about 5 to about 200, more preferably from about 5 to about 80, and wherein for each $R^1$, $R^2$ pair, one is hydrogen and the other is a methyl group. Formulas (V) through (VII) are oversimplified in that, in practice, the orientation of the isopropylene radicals within the B block will be random. This random orientation is indicated in formula (VIII), which is more complete. Such poly (oxyethylene)poly(oxypropylene) compounds have been described by Santon, *Am. Perfumer Cosmet.*, 72(4): 54-58 (1958); Schmolka, *Loc. cit.* 82(7):25-30 (1967); *Non-ionic Surfactants*, Schick, ed. (Dekker, N.Y., 1967), pp. 300-371. A number of such compounds are commercially available under such generic trade names as "lipoloxamers", "pluronics" and "synperonics." Pluronic polymers within the B-A-B formula are often referred to as "reversed" pluronics, "pluronic R" or "meroxapol."

The "polyoxamine" polymer of formula (VIII) is available from BASF (Wyandotte, MI) under the tradename TETRONIC®. The order of the polyoxyethylene and polyoxypropylene blocks represented in formula (VIII) can be reversed, creating TETRONIC-R®, also available from BASF. See, Schmolka, *J. Am. Oil. Soc.,* 59: 110 (1979). Polyoxypropylene-polyoxyethylene block copolymers can also be designed with hydrophilic blocks comprising a random mix of ethylene oxide and propylene oxide repeating units. To maintain the hydrophilic character of the block, ethylene oxide will predominate. Similarly, the hydrophobic block can be a mixture of ethylene oxide and propylene oxide repeating units. Such block copolymers are available from BASF under the trade name PLURADOT®.

Also included is a POE-POP (poly(oxyethylene)-poly(oxypropylene) block copolymer having the formula:

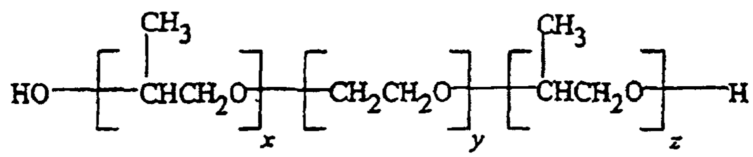

(VII)

in which *x, y,* and *z* have values from about 2 to about 400.

The hydrophobic/hydrophilic properties of a given block copolymer depends upon the ratio of the number of oxyethylene groups to the number of oxypropylene groups. For a composition containing a single block copolymer of poly(oxyethylene)-poly(oxypropylene), for example, this relationship, taking into account the molecular masses of the central hydrophobic block and the terminal hydrophilic blocks, can be expressed as follows:

$$n=\frac{H}{L}\cdot 1.32$$

in which *H* is the number of oxypropylene units and *L* is the number of oxyethylene units. In the general case of a block copolymer containing hydrophobic B-type segments and hydrophilic A-type segments, the hydrophobic-hydrophilic properties and micelle-forming properties are related to the value *n* as defined as:

$$n = (|B|/|A|) \times (b/a)$$

where |B| and |A| are the number of repeating units in the hydrophobic and hydrophilic blocks of the copolymer, respectively, and *b* and *a* are the molecular weights for the respective repeating units.

Selecting a block copolymer with the appropriate *n* value depends upon the hydrophobic/hydrophilic properties of the specific agent, or the composite hydrophilic/hydrophilic properties of a mixture of agents to be formulated. Typically, *n* will range in value from about 0.2 to about 9.0, more preferably between about 0.25 and about 1.5. This range should be viewed not as numerically critical but as expressing the optimum hydrophobic/hydrophilic balance between the predominantly hydrophilic poly(oxyethylene) blocks, and the predominantly hydrophobic poly(oxypropylene) blocks.

A number of pluronics are designed to meet the following formula:

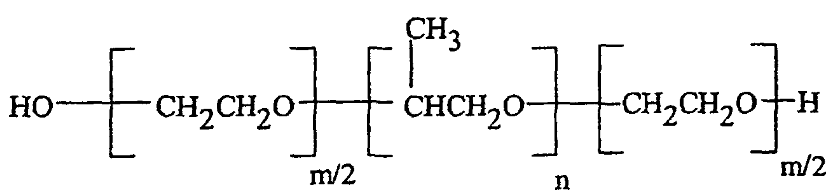

(X)

The characteristics of a number of pluronics, described with reference to formula (X), are as follows:

| Copolymer | Hydrophobe weight | CMC (% w/v) | Hydrophobe percentage |
|---|---|---|---|
| PLURONIC® L61 | 1750 | 0.0003 | 90 |
| PLURONIC® L64 | 1750 | 0.002 | 60 |
| PLURONIC® F68 | 1750 | 4-5 | 20 |
| PLURONIC® P8S | 2250 | 0.005 - 0.007 | 50 |
| PLURONIC® F127 | 4000 | 0.003 - 0.005 | 30 |
| PLURONIC® F108 | 3250 | 0.0035 - 0.007 | 20 |

These CMC values were determined by the surface tension method described in Kabanov *et al*., *Macromolecules* 28: 2303-14 (1995).

Additional specific poly(oxyethylene)-poly(oxypropylene) block copolymers relevant to the invention include:

| PLURONIC | Hydrophobe Weight | Hydrophobe Percentage |
|---|---|---|
| L31 | 950 | 90% |
| F35 | 950 | 50% |
| L42 | 1200 | 80% |
| L43 | 1200 | 70% |
| L44 | 1200 | 60% |
| L61 | 1750 | 90% |
| L62 | 1750 | 80% |
| L63 | 1750 | 70% |
| L64 | 1750 | 60% |
| P65 | 1750 | 50% |
| F68 | 1750 | 20% |
| P75 | 2050 | 50% |
| L81 | 2250 | 90% |
| P84 | 2250 | 60% |
| P85 | 2250 | 50% |
| F87 | 2250 | 30% |
| F88 | 2250 | 20% |
| L92 | 2750 | 80% |
| F98 | 2750 | 20% |
| L101 | 3250 | 90% |
| P103 | 3250 | 70% |
| P104 | 3250 | 60% |
| P105 | 3250 | 50% |
| F108 | 3250 | 20% |
| L121 | 4000 | 90% |
| L122 | 4000 | 80% |
| L123 | 4000 | 70% |
| F127 | 4000 | 30% |
| 10R5 | 1000 | 50% |
| 10R8 | 1000 | 20% |
| 12R3 | 1200 | 70% |
| 17R2 | 1700 | 80% |
| 17R1 | 1700 | 90% |
| 17R2 | 1700 | 80% |
| 17R4 | 1700 | 60% |
| 17R8 | 1700 | 20% |

(continued)

| PLURONIC | Hydrophobe Weight | Hydrophobe Percentage |
|---|---|---|
| 22R4 | 2200 | 60% |
| 25R1 | 2500 | 90% |
| 25R2 | 2500 | 80% |
| 25R4 | 2500 | 60% |
| 25R5 | 2500 | 50% |
| 25R8 | 2500 | 50% |
| 31R1 | 3100 | 90% |
| 31R2 | 3100 | 80% |
| 31R4 | 3100 | 60% |
| *All copolymers above this conform to formula (X), this copolymer and those below conform to formula (VII). | | |

These hydrophobe percentage and hydrophobe weight increases also correlate with improved micelle formation properties wherein micelle formation for these copolymers occurs at lower concentrations. See, Hunter *et al.*, *Macromolecules* 26: 5030 (1993); Hunter *et. al.*, *Macromolecules* 26: 5592 (1993); Alexandris *et. al.*, *Macromolecules* 27: 2414 (1994).

The diamine-linked pluronic of formula (VIII) can also be a member of the family of diamine-linked polyoxyethylene-polyoxypropylene polymers of formula:

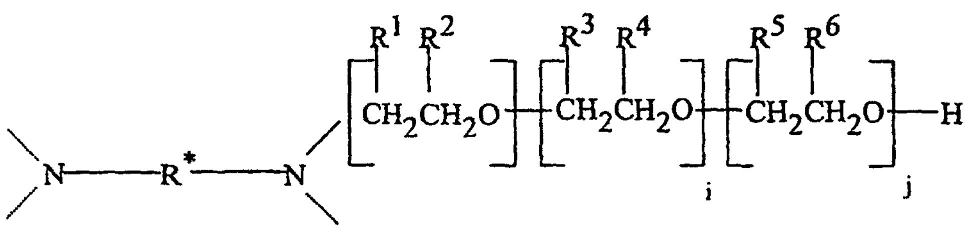

**(XI)**

wherein the dashed lines represent symmetrical copies of the polyether extending off the second nitrogen, R* an alkylene of about 2 to about 6 carbons, a cycloalkylene of about 5 to about 8 carbons or phenylene, for $R^1$ and $R^2$, either (a) both are hydrogen or (b) one is hydrogen and the other is methyl, for $R^3$ and $R^4$ either (a) both are hydrogen or (b) one is hydrogen and the other is methyl, if both of $R^3$ and $R^4$ are hydrogen, then one $R^5$ and $R^6$ is hydrogen and the other is methyl, and if one of $R^3$ and $R^4$ is methyl, then both of $R^5$ and $R^6$ are hydrogen. The $-NH_2-CH_2CH_2-NH_2-$ group of formula (VIII) and the N-R*-N group of formula (X) are examples of linking groups, *L*, of formula (IV).

The Hansch-Leo estimate of the octanol-water partitioning coefficient (P) for an organic molecule is calculated by the following formula:

$$\text{Log P} = a_n f_n + \Sigma b_m F_m$$

where the $f_n$ values are the fragmental constants for the different groups in the molecule, the an values are the number of any type of group in the molecule, the FM values are factors for certain molecular features such as single bonds or double bonds, and the bm values are the number of any such molecular feature. For instance, the Hansch-Leo fragmental constant for an ethylene oxide repeating unit ($-CH_2CHO-$) would be:

$$2f_c + 4f_H + f_0 + (4\text{-}1)F_b = 2(0.20) + 4(0.23) + (-1.82) + 3(-0.12) = -0.86$$

The Hansch-Leo fragmental constant for a propylene oxide ($-CH_2CH(CH_3)O-$) repeating unit would be:

$$2f_c + f_{CH3} + 3fH + f_o + (4\text{-}1)F_b = 2(0.2) + 0.89 + 3(0.23) + (-1.82) + 3(-O.12) = -0.2$$

Those of ordinary skill in the art will recognize that the Hansch-Leo approach to estimating partition constants, in which approach the Hansch-Leo fragmental constants are applied, does not yield precisely the empirical partition constant. See Hansch and Leo, *Substituent Constants for Correlation Analysis in Chemistry and Biology, Wiley, New York*, 1979; James, *Solubility and Related Properties, Marcel Dekker, New York,* 1986, pp. 320-325. However, the approach is precise enough to define the hydrophobicity features of the polymeric delivery vehicle.

The other surfactants that can replace PEG in TPGS can also have the hydrophylic part attached with fatty acids and derivatives thereof, fatty acid soaps including salts of saturated and unsaturated fatty acids and derivatives (*e.g.*, adrenic acid, arachidonic acid, 2-octenoie acid, octanoic acid, nonanoic acid, decanoic acid, undecanoic acid, undecelenic acid, lauric acid, myristoleic acid, myristic acid, palmitic acid, palmitoleic acid, heptadecanoic acid, stearic acid, nonanedecanoic acid, henelcosanoic acid, docasanoic acid, tricosanoic acid, tetracosanoic acid, *cis*-15-tetracosenoic acid, hexacosanoic acid, heptacosanoic acid, octecosanoic acid, triocantanoic acid), salts of hydroxy-, hydroperoxy-, polyhydroxy-, epoxy-fatty acids (see for example, Ingram and Brash, *Lipids*, 1988, 23: 340; Honn *et al.*, *Prostaglandins*, 1992, 44: 413; Yamamoto, Free Radic, *Biol. Med.,* 1991, 10: 149; Fitzpatrick and Murphy, *Pharmacol Rev.,* 1989, 40: 229; Muller *et al.*, *Prostaglandins*, 1989, 38: 635; Falgueyret *et al.*, *FEBS Lett.*, 1990, 262: 197; Cayman Chemical Co., 1994 Catalog, pp. 78-108).

Also included are residues of carboxylic acids for making derivatives of TPGS (*e.g.*, valeric acid, trans-2,4-pentadionoic acid, hexanoic acid, *trans*-2-hexenoic acid, *trans*-3-hexenoic acid, 2,6-heptadienoic acid, 6-heptenoic acid, heptanoic acid, pimelic acid, suberic acid, sebacicic acid, azelaic acid, undecanedioic acid, decanedicarboxylic acid, undecanedicarboxylic acid, dodecanedicarboxylic acid, hexadecanedioic acid, docasenedioic acid, tetracosanedioic acid, prostanoic acid and its derivatives (*e.g.*, Prostaglandins) (see, for example. Nelson *et al.*, *C&EN* 1982, 30-44; Frolich, *Prostaglandins*, 1984, 27: 349; Cayman Chemical Co., 1994 Catalog, pp. 26-61), leukotrienes and lipoxines (see for example, Sarnuelsson *et al.*, *Science*, 1987, 237: 1171; Cayman Chemical Co., 1994 Catalog, pp. 64-76), alkyl phosphates, O-phosphates (*e.g.*, benfotiamine), alkyl phosphonates, natural and synthetic lipids (*e.g.*, dimethylallyl pyrophosphate-ammonium salt, Sfemesylthioacetic acid, famesyl pyrophphosphate, 2-hydroxymyristic acid, 2-fluoropalmitic acid, inositoltriphosphates, geranyl pyrophosphate, geranygeranyl pyrophosphate, α-hydroxyfamesyl phosphonic acid, isopentyl pyrophoshate, phosphatidylsorines, cardiolipines, phosphatidic acid and derivatives, lysophosphatidic acids, sphingolipids and the like), synthetic analogs oflipids such as sodium-dialkyl sulfosuccinate (*e.g.*, Aerosol OT®), n-alkyl ethoxylated sulfates, n-alkyl monothiocarbonates, alkyl- and aryisulfates (asaprol, azosulfamide, *p*-(benzyl-sulfonamideo)benzoic acid, cefonicid, CHAPS), mono- and dialkyl dithiophosphates, N-alkanoyl-N-methylglucamine, perfluoroalcanoate, cholate and desoxychoate salts of bile acids, 4-chloroindoleacetic acid, cucurbic acid, jasmonic acid, 7-epi jasmonic acid, 12-oxo-phytodienoic acid, traumatic acid, tuberonic acid, abscisic acid, acitertin, and the like. The carboxylic acid derivatives are useful for attachment of targeting moieties to the TPGS derivatives.

The hydrophobic part can also be attached by long alkyl chain amines including primary, secondary and tertiary amines (*e.g.*, hexylamine, heptylamine, octylamine, decylamine, undecylamine, dodecylamine, pentadecyl amine, hexadecyl amine, oleylamine, stearylamine, diaminopropane, diaminobutane, diaminopentane, diaminohexane, diaminoheptane, diaminooctane, diaminononane, diaminodecane, diaminododecanery amines, N,N-distearylamine, N,N',N'-polyoxyethylene(10)-N-tallow-1,3-diaminopropane).

In a specific embodiment of the present invention, the tocoferol covalently linked to a water-soluble polymer is a 'TPGS-peptide' derivative made by using a linker, for example, but not limited to, 3(2-pyridyl dithio) propionic acid N-hydroxysuccinimide ester, and by attaching a variety of peptides on this linker. Any peptide or protein that is hydrophilic or amphiphilic can be used to make the TPGS-peptide derivatives so that the TPGS-peptide derivative forms micelles.

## MICELLE FORMATION

In the present invention the compositions comprise micelles. The importance of the micellar form in delivering biological agents is also revealed in *in vivo* experiments. In micellar form, biological agents are located in the hydrophobic core of the micelles, thereby masked by the hydropbilic shell (composed of polyethylene glycol residues) surrounding the micelles. This masking decreases interactions with liver, plasma proteins, other non-target tissues and other molecules that can bind or inactivate the agent or convert the agent to a toxic metabolite.). Using doxorubicin as

a model drug, the micellar form decreases liver uptake, decreases conversion to doxorubicinol, and decreases the rate at which the concentration of doxorubicin decreases in the blood.

The effectiveness of surfactant molecules in (a) forming micelles (where greater effectiveness is measured in reduced CMC) and (b) favoring the partitioning of various biological agents to the micellar rather than the free form of various biological agents increases according to the same pattern. The presence of micelles at low concentrations is believed to help assure, assuming that biological agent remains associated with the micelles, that the biological agent and the copolymer arrive together at a target tissue. Partitioning coefficients that favor the micellar form help assure that the assumption that the biological agent remains associated with micelles will hold true. The micellar form of the biological agent is also believed to protect the biological agent from uptake by non-target tissues, which tissues may metabolize the biological agent into an ineffective or toxic metabolite, and nonspecific adsorption to blood components, cellular components and the like.

The small size of the micelles formed by according to the present invention is believed to allow these micelles to penetrate in small capillaries and to be taken up by cells.

According to the present invention the tocopherols covalently linked to a water-soluble polymer form colloidal dispersions (including suspensions, emulsions, microemulsions micelles, polymer complexes, or other types of molecular aggregates). The size of the species formed is one major parameter determining usefulness of the compositions of the current invention. After administration in the body large particles are eliminated by the reticuloendothelial system and cannot be easily transported to the disease site (see, for example, Kabanov *et al*., *J. Contr. Release*, 22, 141 (1992); *Volkheimer. Pathologe* 14:247 (1993); Kwon and Kataoka, *Adv. Drug. Del. Rev.* 16:295 (1995). Also, the transport of large particles in the cell and intracellular delivery is limited or insignificant. See, *e.g*., Labhasetwar et al. *Adv. Drug Del. Res*. 24:63 (1997). It was demonstrated that aggregated cationic species with a size from 300 nm to over 1 µm are ineffective in cell transfection, see Kabanov *et al*., Self-Assembling Complexes for Gene Delivery, from Laboratory to Clinical Trial, Kabanov et al. (eds.), John Wiley, Chichester (1998) and references cited. Large particles, particularly, those positively charged exhibit high toxicity in the body, in part due to adverse effects on liver and embolism. See e.g., *Volkheimer, Pathologe* 14:247 (1993); *Khopade et al Pharmazie* 51:558 (1996); Yamashita *et al*., *Vet. Hum. Toxicol*., 39:71 (1997). Small surfactant species are nontoxic, can enter into small capillaries in the body, transport in the body to a disease site, cross biological barriers (including but not limited to the blood-brain barrier and intestinal epithelium), absorb into cell endocytic vesicles, cross cell membranes and transport to the target site inside the cell. The particles in that size range are believed to be more efficiently transferred across the arterial wall compared to larger size microparticles, see *Labhasetwar et al*., *Adv. Drug Del. Res.* 24:63 (1997). Without wishing to be bound by any particular theory it is also believed that because of high surface to volume ratio, the small size is essential for successful targeting of such particles using targeting molecules. The preferred range of the species formed in the compositions of the current invention is less than 300 nm, more preferred less than 100 nm, still more preferred less than 50 nm.

Typically the micelles will have an average diameter of from 3 to 25 nm, although this range can vary widely. The average diameter of any given preparation can be readily determined by quasi-elastic light scattering techniques.

The compositions of the invention are intended to include either preformed micelles with a substantial portion of the biological agent incorporated therein, or copolymer compositions which form micelles with a substantial portion of the agent dissolved therein during the course of the administration of the biological agent to a patient, or subsequent thereto. For the targeting embodiment of the invention, the targeting moiety will either be pre-associated with micelles or will associate with micelles during the course of administration.

## TARGETING MOLECULES

In another embodiment, the invention compositions as defined above, which further comprise a targeting molecule or moiety. Preferably, the targeting molecule comprises a targeting moiety and a lipophilic moiety. More preferably, the targeting moiety is an antibody, hormone, carbohydrate, drug, cytokine, interleukin or peptide. It is often desirable to target a biological agent to a particular tissue in which the agent is anticipated to beneficially act. This desirability is particularly true for chemotherapeutic agents that potentially have highly toxic effects on non-target tissues. Most anti-cancer chemotherapeutic agents function by selectively poisoning replicating cells. This mechanism inevitably targets the rapidly replicating cells, such as those of the bone marrow which generate a number of important blood cells. If the biodistribution of the chemotherapeutic drug is changed so that useful concentrations are maintained in the cancerous tissue or the tissue in which the cancer resides while concentrations distal from the cancer *situs* are reduced, the scope of toxic side effects will generally be reduced.

The targeting molecules comprise a targeting moiety which is coupled to a lipophilic moiety comprising a hydrocarbon having from about 3 to about 41 carbon atoms. This targeting moiety is incorporated into the micelles of the compositions of the targeting embodiment This portion of the targeting molecule typically comprises no more than about 10% w/w of the copolymer components of a composition. The lipophilic moieties are believed to act as hydrophobic "anchors", which are incorporated non-covalently into the block-copolymer micelles so that the targeting moiety becomes part of, but extends beyond, the micelle.

The targeting moieties have affinity for a cellular, tissue, viral or substratum site. Typical targeting moieties include, but are not limited to, antibodies and hormones with affinity for a cellular binding component, any molecule containing a carbohydrate moiety recognized by a cellular binding component, and drugs that bind to a cellular binding component. The phrase "binding component" includes both receptor and acceptor molecules. Preferably, the binding component is a cell-surface binding component. Both polyclonal and monoclonal antibodies which are either available commercially or described in the literature can be employed. Alternatively the targeting moiety is a naturally occurring protein, such as insulin, that binds to a target site. A non-limiting example of a targeting moiety is the anti-$\alpha_2$-GP antibody to brain glial cells ($\alpha_2$-glycoprotein) which is described by Slepnev *et al.*, *Bioconjugate Chem.,* 3: 273-274 (1992).

As mentioned above, the targeting moieties can have affinity for a cellular, tissue, viral or substratum site. The targeting moiety can be a peptide including antibodies for example, but not limited to an anti-$\alpha_2$-P antibody specific for the $\alpha_2$-glycoprotein of glial cell, an anti-GFAP antibody, a monoclonal antibody against the neuronal-specific enolase. Also included are a variety of human and animal cytokines including interferons as human natural and recombinant interferon-$\alpha_2$, interleukins such as human recombinant Interleukin-2 (IL-2), tumor necrosis factors (TNFs) such as human recombinant TNF-$\alpha$ and a number of other proteins and peptides which are factors controlling the immune system. Targeting moieties can also be hormones such as insulin.

In another specific embodiment of the present invention, the targeting moiety is a synthetic peptide that binds to a cell which is identified from a peptide library. Screening methods and peptide libraries are known to those skilled in the art of peptide chemistry, and synthetic peptides with binding affinities are easily identified using commonly known methods of screening and binding assays.

## METHODS OF USE

The present invention also provides the use of the compositions of the invention in the manufacture of a medicament for inhibiting cancer. The invention also provides processes for delivering to a cell, *ex vivo*, a composition of the invention as defined above.

In one embodiment, the composition further comprises a targeting molecule. In a more specific embodiment, the tocoferol covalently linked to a water-soluble polymer of the administered compositions is TPGS or a derivative thereof.

The compositions of the present invention can be used for treatment of animals, including, but not limited to animals such as chickens, pigs, cows, cats, dogs, horses, fish, shrimp, and preferably to mammals, and most preferably humans.

The present compositions can be used in a variety of treatments. The compositions and methods of treatment of the present invention can be used to treat a variety of diseases or conditions such as, but not limited to diseases of the liver and kidneys and for scrofula, syphilis, gonorrhea, coughs, genital warts, cancers, herpes simplex type-1 and other viral agents. The compositions can also be used for insecticides, herbicides and as ichthytoxins.

In a specific embodiment, the method comprises administering a composition that further comprises a targeting molecule. In another specific embodiment, the tocoferol covalently linked to a water-soluble polymer of the the administered composition is a TPGS or derivative thereof.

The method of inhibiting cancer can be applied to cancers such as, but not limited to, oat carcinoma of the lung, ovarian cancer, mall cell bronchus carcinoma, testicular carcinoma, choriocarcinoma and neuroblastoma, myelocytic and monocytic malignancies in children, lymphocyte leukemia, myelogenous leukemia, Hodgkin's diseases, histocytic lymphoma, Wilm's tumor, Ewing's sarcoma, sarcoccygeal sarcoma, and acute leukemia. The compositions can be used to treat any cancers for which podophyllotoxins, analogs thereof or derivatives thereof are currently known or are eventually shown to have activity against.

## METHODS OF ADMINISTRATION

The compositions of the present invention can be administered by a number of routes, including without limitation, orally, topically, rectally, vaginally, by pulmonary route for instance by use of an aerosol, or parenterally, including but not limited to intramuscularly, intradermally, subcutaneously, intraperitoneally, intra-arterially or intravenously. The compositions can be administered alone, or can be combined with a pharmaceutically acceptable carrier or excipient according to standard pharmaceutical practice.

For oral administration, the compositions can be used in the form of tablets capsules, lozenges, troches, powders, syrups, elixirs, aqueous solutions and suspensions, and the like. In the case of tablets, carriers that can be used include lactose, sodium citrate and salts of phosphoric acid. Various disintegrants such as starch, and lubricating

agents such as magnesium stearate, sodium lauryl sulfate and talc, are commonly used in tablets. For oral administration in capsule form, useful diluents are lactose and high molecular weight polyethylene glycols. When aqueous suspensions are required for oral use, the compositions can be combined with emulsifying and suspending agents. If desired, certain sweetening and/or flavoring agents can be added. For parenteral administration, sterile solutions of the conjugate are usually prepared, and the pHs of the solutions are suitably adjusted and buffered.

For intravenous use, the total concentration of solutes should be controlled to render the preparation isotonic. For ocular administration, ointments or droppable liquids may be delivered by ocular delivery systems known to the art such as applicators or eyedroppers. Such compositions can include mucomimetics such as hyaluronic acid, chondroitin sulfate, hydroxypropyl methylcellulose or poly(vinyl alcohol), preservatives such as sorbic acid, EDTA or benzylchronium chloride, and the usual quantities of diluents and/or carriers.

For pulmonary administration, diluents and/or carriers will be selected to be appropriate to allow the formation of an aerosol.

Suppository forms of the compositions of the invention are useful for vaginal, urethral and rectal administrations. Such suppositories will generally be constructed of a mixture of substances that is solid at room temperature but melts at body temperature. The substances commonly used to create such vehicles include theobroma oil, glycerinated gelatin, hydrogenated vegetable oils, mixtures of polyethylene glycol of various molecular weights and fatty acid esters of polyethylene glycol. *See Remington's Pharmaceutical Sciences, 16th Ed.*, *Mack Publishing, Easton, PA,* 1980, pp. 1530-1533 for further discussion of suppository dosage forms. Analogous gels or creams can be used for vaginal, urethral and rectal administrations.

In the present invention the formulation of the podophyllotoxin derivative is a less complex mixture since it comprises a chemotherapeutic agent and its solubilizing compound which are in a micellar form in an aqueous isotonic medium. Avoiding the use of organic solvents may prevent inducing hypersensitivity reactions observed previously. In addition, for lymphomas and small-cell lung cancer, which have a high frequency of metastases to the brain or meninges, an intrathecal therapy may be indicated. The absence of organic solvents in the present formulation allows the use of this route of administration to be considered. Additionally, the preferred solubilizing component used in this formulation has been recognized as very safe and not responsible for toxic side effects (See Eastman Chemical Company for *Vitamin E TPGS Drug Master Files and General Recognition of Safety (GRAS) Data Sheets).*

The dosage for a podophylotoxin in a micellar composition will often be about that of the podophyllotoxin alone. Dosages will be determined by the prescribing medical professional considering many factors including the patients age, weight and condition and the pharmacokinetics of the agent. Dosages are routinely determined by those of skill in the art. However, the amount of a micellar form of an agent required for effective treatment may often be less than the amount required using the free biological agent. For instance, a typical intravenous dose of etoposide will be 450-900 mg in an adult patient weighing 70 kg over a period of 3-5 days.

The podophyllotoxins used in the invention are administered to an animal in an effective amount. The effect of the tocoferol covalently linked to a water-soluble polymer or TGPS or derivative thereof used in the composition on the drug's effectiveness must be considered in determining the effective amount. Generally, an effective amount is an amount effective to either: (1) reduce the symptoms of the disease sought to be treated or (2) induce a pharmacological change relevant to treating the disease sought to be treated. For cancer, an effective amount includes an amount effective to reduce the size of a tumor; slow the growth of a tumor, prevent or inhibit metastases; or increase the life expectancy of the affected animal.

<u>METHODS OF DELIVER TO CELLS</u>

An embodiment of the present invention is a process of delivering, *ex vivo*, a composition of the invention to a cell comprising the administration thereof to a cell.

In a more specific embodiment, the tocoferol covalently linked to a water-soluble polymer used in the composition administered to the cell is TPGS or a derivative thereof.

In a specific embodiment, the method of delivering the podophyllotoxin to a cell comprises administering a composition of the invention which further comprises a targeting molecule.

Target cells for the delivery of the podophyllotoxin composition are, but not limited to, procaryotic or eucaryotic cells, preferably animal cells, more preferably mammalian cells, and most preferably human cells. Cell targets can be *ex vivo* and/or *in vivo*, and include T and B lymphocytes, primary CML, tumor infiltrating lymphocytes, tumor cells, leukemic cells (such as HL-60, ML-3, KG-1 and the like), skin fibroblasts, myoblasts, cells of central nervous system including primary neurons, liver cells, carcinoma (such as Bladder carcinoma T24, human colorectal carcinoma Caco-2), melanoma, CD34+ lymphocytes, NK cells, macrophages, hemotopoetic cells, neuroblastona (such as LAN-5 and the like), gliomas, lymphomas (such as Burkitt lymphomas ST486), JD38), T-cell hybridomas, muscle cells such as primary smooth muscle, and the like.

EXAMPLE 1: Preparation of Etoposide Formulation (Formulation A)

Ten (10) mg of Etoposide dissolved in 1mL of methanol were added to 200mg TPGS dissolved in 1mL of methanol. The methanol was evaporated to dryness using a speed-vac concentrator. The residue was mixed with 5 ml of phosphate buffer saline (PBS) pH 5.5, and the mixture was shaken gently for complete dispersion at room temperature for 30 min. The final formulation, containing 2 mg/ml of Etoposide and 40 mg/ml *i.e.* 4% of TPGS, was sterilized for intravenous administration by passing it through a 0.2 µm filter. The final solution consists of a clear and transparent liquid slightly yellowish in color. The formulation did not show any precipitation of the drug when stored at room temperature for at least 36 hours.

EXAMPLE 2: Solubility of Etoposide in Formulation A

A stock solution of Etoposide (10 mg/ml methanol) and a stock solution of TPGS (100 mg/ ml methanol) were prepared. To 100 µl of Etoposide aliquots was added an aliquot of TPGS solution to obtain the range of final concentration: 0, 1, 2, 3, 4, 5, 6, 7, 8, 9 and 10% TPGS. At each concentration the samples were prepared in duplicate. The methanol was then evaporated as described in Example 1. The residue was added to 100 µl of PBS and the mixture was shaken for complete dispersion at room temperature. An aliquot (50 µl) of each sample was extracted with 450 µl of acetonitrile. The organic phase was then released from the extraction solution by adding about 20 mg of NaCl. After centrifugation at 10,000 rpm for 10 min, the organic phase containing Etoposide was separated and evaporated to dryness under a stream of nitrogen at 60°C. The dry extracts obtained were dissolved in 1 ml of "mobile phase" solution of 25% buffer A (1% acetic acid in acetonitrile)/75% buffer B (1% acetic acid in water). Finally the samples were analyzed for Etoposide concentration using reverse phase HPLC with fluorescence detection. Separation was achieved using a $C_{18}$ column (90 Å; 250 x 4.6 mm) at 32°C and a "mobile phase" consisting of a solution of 25% buffer A (1% acetic acid in acetonitrile) / 75% buffer B (1% acetic acid in water) at a flow rate of 1.0 ml/min. The excitation and emission wavelengths were set at 230 and 323 nm, respectively. The amount of Etoposide in each sample was calculated from the area under the peak (AUP) using a calibration curve for Etoposide.

The data for maximal solubility of Etoposide at room temperature are set forth in Table 1 (each value was expressed as a mean ± standard deviation). The results demonstrate that etoposide is increasingly soluble in solutions of increasing concentrations of TPGS.

Table 1.

| Etoposide solubility in TPGS solutions. | | | | | | | | | | |
|---|---|---|---|---|---|---|---|---|---|---|
| TPGS % | 1% | 2% | 3% | 4% | 5% | 6% | 7% | 8% | 9% | 10% |
| Etoposide solubility (mg/ml) | 1.3 ± 0.15 | 1.6 ± 0.2 | 1.75 ± 0.3 | 2.0 ± 0.15 | 2.1 ± 0.6 | 3.0 ± 0.9 | 4.7 ± 0.35 | 6.0 ± 0.4 | 7.7 ± 0.4 | 8.7 ± 0.9 |

EXAMPLE 3: Thermodynamic Stability of Etoposide in Formulation A

Loss of thermodynamic stability of Etoposide was evaluated at room temperature by the appearance of a fine white precipitate in the formulation. The samples were prepared according to Example 1. Visual inspection was performed after centrifugation of the sample over different periods of time. The results obtained are shown below in Table 2 (Etoposide stability is indicated by grey shading). The results demonstrate that etoposide is stable for longer periods of time in solutions of increasing concentration of TGPS.

Table 2. Etoposide stability after storage at room temperature

| Storage time (hours) | | 1 | 3 | 6 | 24 | 36 | 48 | 72 | 96 |
|---|---|---|---|---|---|---|---|---|---|
| 1% TPGS | | | | | | | | | |
| Etoposide (mg/mL) | 0.5 | | | | | | | | |
| | 1.0 | | | | | | | | |
| | 1.5 | | | | | | | | |
| | 2.0 | | | | | | | | |
| 2% TPGS | | | | | | | | | |
| Etoposide (mg/mL) | 0.5 | | | | | | | | |
| | 1.0 | | | | | | | | |
| | 1.5 | | | | | | | | |
| | 2.0 | | | | | | | | |
| 3% TPGS | | | | | | | | | |
| Etoposide (mg/mL) | 0.5 | | | | | | | | |
| | 1.0 | | | | | | | | |
| | 1.5 | | | | | | | | |
| | 2.0 | | | | | | | | |
| 4% TPGS | | | | | | | | | |
| Etoposide (mg/mL) | 0.5 | | | | | | | | |
| | 1.0 | | | | | | | | |
| | 1.5 | | | | | | | | |
| | 2.0 | | | | | | | | |

EXAMPLE 4: Preparation of an Etoposide Formulation (Formulation B)

One (1) mg of Etoposide was disolved in 100 µl of acetonitrile. Then 100 µl of 10% solution of a PLURONIC® P85 in acetonitrile and 20 µl of a 3 M sodium acetate in water were added to the Etoposide solution. The mixture was evaporated to dryness using a speed-vac concentrator. One (1) ml of phosphate buffer saline (PBS) was added to the residue and the mixture was dissolved using a rotator. PLURONIC® P85 was not suitable to solubilize etoposide successfully. Formulation B was unstable since a drug precipitate formed within 30 min at room temperature.

EXAMPLE 5: Preparation of an Etoposide Formulation (Formulation C)

One (1) mg of Etoposide was dissolved in 100 µl of acetonitrile. Then 100 µl of 10% solution of PLURONIC® P85 in acetonitrile, 200 µl of a 10% solution of Pluronic F127 in acetonitrile, 50 µl of a 5% solution of PLURONIC® L61 and 10 µl of a 3 M sodium acetate in water were added to the Etoposide solution. The mixture was evaporated to

dryness using a speed-vac concentrator. One (1) ml of phosphate buffer saline (PBS) was added to the residue, and the mixture was dissolved using a rotator. Formulation C was unstable since a drug precipitate formed rapidly at room temperature.

EXAMPLE 6: Preparation of an Etoposide Formulation (Formulation D)

Four (4) mg of Etoposide was dissolved in 200 µl of acetonitrile. Then 100 µl of 10% solution of PLURONIC® P85 in acetonitrile, 20 µl of 15 mM sodium salt of nonafluoropentanoic acid (NFPNa) solution in ethanol and 5 µl of 3M sodium acetate in water were added to the Etoposide solution. The mixture was evaporated to dryness using a speed-vac concentrator. One (1) ml of 2% solution of PLURONIC® F127 in PBS was added to the residue and the mixture was dissolved using a rotator. The use of PLURONIC® P85 and P127 was not satisfactory since the final solution formed a drug precipitation in less of 30 minutes at room temperature.

EXAMPLE 7: Preparation of an Etoposide Formulation (Formulation E)

Three (3) mg of Etoposide was dissolved in 100 µl of acetonitrile. Then 100µl of a 10% solution of PLURONIC® P85 in acetonitrile, 20 µl of 15 mM sodium salt of nonafluoropentanoic acid (NFPNa) solution in ethanol, and 5 µl of 3 M sodium acetate in water were added to the Etoposide solution. The mixture was evaporated to dryness using a speed-vac concentrator. One (1) ml of PBS was added to the residue and the mixture was dissolved using a rotator. Formulation E was very unstable since a drug precipitate appeared in less than 30 minutes.

EXAMPLE 8: Preparation of an Etoposide Formulation (Formulation F)

Three (3) mg of Etoposide was dissolved in 150 µl of a methanol/acetonitrile (2:1) mixture. Twenty (20)µl of 15 mM sodium salt of nonafluoropentanoic acid (NFPNa) solution in methanol and 5 µl of 3M sodium acetate in water were added to the Etoposide solution. The mixture was evaporated to dryness using a speed-vac concentrator. The residue was dissolved in 1 ml of 2% solution of PLURONIC® F127 in PBS using a rotator. Formulation F was unstable since a drug precipitate formed gradually within 1 hour at room temperature.

EXAMPLE 9: Preparation of an Etoposide Formulation (Formulation G)

Two (2) mg of Etoposide was dissolved in 150 µl of a methanol/acetonitrile (2:1) mixture. Two hundred (200) µl of 10% solution of PLURONIC® P104 in methanol was added to the Etoposide solution. The mixture was evaporated to dryness using a speed-vac concentrator. The residue was dissolved in 1 ml of PBS using a rotator. Formulation G was unstable since a drug precipitation occurred gradually within 1 hour at room temperature.

EXAMPLE 10: Preparation of an Etoposide Formulation (Formulation H)

Two (2) mg of Etoposide was dissolved in 150 µl of a methanol/acetonitrile (2:1) mixture. Then 200 µl of a 10% solution of PLURONIC® P 104 in methanol, and 100 µl of 10% solution of PLURONIC® F108 in methanol were added to the Etoposide solution. The mixture was evaporated to dryness using a speed-vac concentrator. The residue was dissolved in 1 ml PBS using a rotator. Formulation H was unstable since a white drug precipitate formed almost immediately.

EXAMPLE 11: Preparation of an Etoposide Formulation (Formulation I)

Two (2) mg of Etoposide was dissolved in 150 µl of a methanol/acetonitrile (2:1) mixture. Two hundred (200) µl of a 10% solution of PLUROIVIC® P 104 in methanol, and 100 µl of 10% solution of PLURONIC® F68 in methanol were added to the Etoposide solution. The mixture was evaporated to dryness using a speed-vac concentrator. The residue was dissolved in 1 ml of PBS using a rotator. Formulation I was unstable since it turned cloudy immediately after preparation.

EXAMPLE 12: Preparation of an Etoposide Formulation (Formulation J)

Two (2) mg of Etoposide was dissolved in 150 µl of a methanol/acetonitrile (2:1) mixture. Two hundred (200) µl of a 10% solution of PLURONIC® P104 in methanol and 100 µl of 10% solution of PLURONIC® F87 in methanol were added to the Etoposide solution. The mixture was evaporated to dryness using a speed-vac concentrator. The residue was dissolved in 1 ml of PBS using a rotator. The combination of PLURONIC® P104 and P87 did not lead to

a stable formulation since a net drug precipitate could be visualized within 30 minutes at room temperature.

EXAMPLE 13: Anticancer Activity of Etoposide Formulation A Against Lewis Lung Carcinoma 3LL-M27

C57BL/6 mice (female six/seven-week-old) were intravenously administered either the clinical form of Etoposide (VEPESID®, Bristol-Myers Squibb; each ml containing 20 mg etoposide; 20 mg polysorbate 80; 650 mg polyethylene glycol 300; 30 mg benzyl alcohol; 2 mg citric acid and 1 ml absolute alcohol *qs*) or with the Etoposide of Formulation A.

The animals were injected with 3LL-M27 cells inoculation ($5x10^5$ cells per animal) and were randomly divided into the groups as described below. The Etoposide concentration in Formulation A was 2mg/ml and the injection volume corresponded with the expected doses. VEPESID® was diluted with PBS at concentrations from 20 mg/ml to 1.5 mg/ml. The dose of Etoposide was 15 mg/kg for both formulations. The animals of the control group received PBS alone. Another control group received only the carrier of Formulation A.

Five to six animals in each group were sacrificed on day 13 for routine metastasis inspection (The time to sacrifice animals was decided by monitoring metastasis development in control group). Although all organs were routinely screened, metastasis formation was normally detected only in the lungs. The metastasis colonies on the organ surface were enumerated immediately following the removal of the tissues. The lungs were subsequently fixed in 10% phosphate-buffered formalin for pathological analysis. The animal was considered to have 50 metastasis sites if the number of tumor nodules on the organ surface was equal to or higher than 50 per mouse. The treatment schedule and results are set forth below in Tables 3 and 4.

Table 3.

| Comparison of the Effects of Etoposide Formulation A and VEPESID® on Lung Metastasis. | | | | |
|---|---|---|---|---|
| Treatment | Treatment Schedule (on day after cell inoculation) | Injection Volume (ml/kg) | Number of Animals | Lung Metastasis Numbers (sample number) on day 13 |
| Control (PBS) | 1, 3, 5, 7 | 10 | 26 | > 50 (5) |
| Formulation A Carrier | 1, 3, 5, 7 | 10 | 26 | > 50 (5) |
| Vepesid (15 mg/kg) | 1, 3, 5, 7 | 10 | 22 | > 50 (5) |
| Formulation A (15 mg/kg) | 1, 3, 5, 7 | 7.5 | 22* | 9.8 ± 4.0 (6)** |

* : Five animals were dead on day 10 to 13 because of treatment toxicity.

**: P < 0.01, Formulation A *vs* Control and VEPEDID®.

Table 4.

| Survival of C57BL/6 Female Mice Implanted with 3LL-M27 Cells After Intravenous Administration of VEPESID® or Formulation A | | |
|---|---|---|
| Treatment | Animal Number for Survival Observation | Median Survival Time (day) |
| Control (PBS) | 18 | 15.2 ± 0.93 |
| Vepesid (15 mg/kg) | 17 | 20.5 ± 0.69 |
| Formulation A (15 mg/kg) | 11 | 25.7 ± 1.3 |

The results demonstrate that Etoposide Formulation A showed greater efficacy against lung metastasis as compared to VEPESID®.

EXAMPLE 14: Anticancer Activity of Etoposide Formulation A Against Lewis Lung Carcinoma

C57BL/6 mice (female six/seven-week-old) were intravenously administered with either the clinical form of Etoposide, (VEPESID®) or with the Etoposide Formulation A using the dose of 15 mg/kg. The protocol is identical to that described in Example 13 except the injections were performed on day 1, 3, and 5 after cell implantation.

The treatment schedule and results are set forth below in Table 5. The results demonstrate that etoposide of Formulation A was more effective against the lung carcinoma that VEPESID.

Table 5

| Etoposide Treatment Schedule and Number of Metastases. | | | | |
|---|---|---|---|---|
| Treatment | Treatment Schedule (on day after cell inoculation) | Injection Volume (ml/kg) | Number of Animals | Lung Metastasis Numbers (sample number) on day 13 |
| Control (PBS) | 1, 3, 5 | 10 | 26 | > 50 (4) |
| VEPESID® (15 mg/kg) | 1, 3, 5 | 10 | 22 | 40.6 ± 5.6 (6)* |
| Formulation A (15 mg/kg) | 1, 3, 5 | 7.5 | 22 | 20.8 ± 8.0 (7)** |

* : $P<0.01$, Vepesid *vs* Control.
**: $P<0.01$, Formulation A *vs* Control, Vepesid.

EXAMPLE 15: Evaluation of Lung Metastasis Development After Intravenous Administration of Etoposide formulation A and VEPESID Against Lewis Lung Carcinoma

C57BL/6 mice (female six/seven week-old)-were intravenously administered with either the clinically used Etoposide (VEPESID®) or the Etoposide Formulation A according to the protocol of Example 14. Metastasis development was monitored on day 14 after 3LL-M27 cells were implantated into the mice.

The categories of response for treatment were evaluated as follows: 'complete' refers to metastasis number less than 5; 'partial' refers to animals bearing 5 to 39 metastasis; and 'progression' to animals whose metastasis number equals or exceeds 40. The categories of responses were expressed as a percentage of the animals bearing the metastasis number. The results are presented in Table 6 below. The results demonstrate that the Etoposide Formulation A of the present invention was more effective in reducing the number of metastases than the clinical VEPESID® formulation.

Table 6

| Response of Metastasis to Etoposide Formulations. | | | | |
|---|---|---|---|---|
| Treatment | Treatment Schedule (on day after cell inoculation) | Response Categories (%) | | |
| | | Complete | Partial | Progression |
| Control (PBS) | 1, 3, 5 | 0 | 0 | 100 |
| Vepesid (15 mg/kg) | 1, 3, 5 | 0 | 50 | 50 |
| Formulation A (15 mg/kg) | 1, 3, 5 1, 3, 5 | 43 43 | 43 43 | 14 14 |

EXAMPLE 16: Pharmacokinetics of Etoposide in Formulation A and VEPESID After Intravenous Administration in Normal Mice

C57BL/6 mice (female six/seven-week-old) were intravenously administered with either the clinical form of Etoposide (VEPESID®) or the Etoposide Formulation A using a single dose of 30mg/kg. Three animals per time point were sacrificed at 5, 30, and 60min, and 3, 6, 10 and 24 hours after injection. Blood was collected and plasma separated. Plasma samples were immediately frozen at -80°C until analysed for Etoposide concentration.

The defrosted plasma samples were separated by centrifugation and aliquots (100 µl) of each sample were extracted with 900 µl of acetonitrile for 15 min on a shaker. Then about 50 mg NaCl were added to each aliquot and the samples were shaken for 5 min and centrifuged at 10,000 rpm for 15 min. The organic phase was transferred, evaporated under a stream of nitrogen at 60°C and reconstituted into 100 µl of mobile phase for analysis. Quantitation of Etoposide in sample aliquots was performed by using a reverse phase HPLC system with fluorescence detection as described previously in Example 2, except that the mobile phase consisted in 23% buffer A (1% acetic acid in acetonitrile) / 77% buffer B (1% acetic acid in water). The amount of Etoposide in each sample was calculated from the area under the peak using a calibration curve for mouse plasma. The area under the curve (AUC) for plasma

etoposide was calculated by trapezoidal integration.

The pharmacokinetic parameters, area under the concentration *vs* time curve (AUC) and mean residence time (MRT), obtained for VEPESID and Etoposide formulation A in mouse plasma are set forth in Table 7. The results demonstrate that more etoposide was present in plasma of mice that received Formulation A than those who received VEPESID.

Table 7

| Concentrations of Etoposide in Plasma Samples | | | | |
|---|---|---|---|---|
| Parameter | Dimension | VEPESID | Formulation A | Ratio A/ VEPESID |
| AUC | (μg x hr)/ml | 30.54 | 85.40 | 2.80 |
| MRT | min | 18.6 | 45.6 | 2.45 |

## Claims

**1.** A composition comprising an aqueous dispersion of micelles having an average diameter of less than 300 nm, said micelles comprising:

(a) a podophyllotoxin selected from etoposide and teniposide and analogues and derivatives thereof; and
(b) tocoferol covalently linked to a water-soluble polymer.

**2.** A composition according to claim 1, wherein the podophyllotoxin is etoposide.

**3.** A composition according to claim 1 or 2, wherein the water-soluble polymer is selected from poly-oxyethylene, poly-oxyethylene-poly-oxypropylene copolymers, polyacrylamides, polyglycerols, polyvinvylalcohols, polyvinylpyrrolidones, polyvinylpyridine N-oxides, copolymers of vinylpyridine N-oxide and vinylpyridine, polyoxazolines, polyacroylmorpholines, and derivatives thereof.

**4.** A composition according to any one of claims 1 to 3, wherein the water-soluble polymer is a polypeptide or derivative thereof.

**5.** A composition according to any one of claims I to 4, wherein the water-soluble polymer further comprises a hydrophobic group other than tocoferol.

**6.** A composition according to any one of claims 1 to 5, wherein the tocoferol covalently linked to a water-soluble polymer is d-α-tocopheryl polyethylene gylcol 1000 succinate (TPGS) or a derivative thereof.

**7.** A composition according to claim 6, wherein the TPGS is present at a concentration of from 0.02 wt % to 20 wt %.

**8.** A composition according to claim 6 wherein the TPGS is present at a concentration of from 0.02 wt % to 10 wt %.

**9.** A composition according to claim 6, wherein the TPGS is present at a concentration of from 4 wt % to 10 wt %.

**10.** A composition according to any one of claims 1 to 9, further comprising a targeting molecule.

**11.** A composition according to claim 10, wherein the targeting molecule comprises a targeting moiety and a lipophilic moiety.

**12.** A composition according to claim 10 or 11, wherein the targeting moiety is an hormone, carbohydrate, drug, cytokine, interlueukin or peptide.

**13.** A composition according to any one of claims 1 to 12, wherein the micelles have an average diameter of less than 100 nm.

**14.** A composition according to any one of claims 1 to 12, wherein the micelles have an average diameter of less than 50 nm.

15. A composition according to any one of claims 1 to 12, wherein the micelles have an average diameter of from 3 nm to 25 nm.

16. A composition as defined in any one of claims 1 to 15 for use as a medicament.

17. A process for delivering to a cell, *ex vivo*, a podophyllotoxin selected from etoposide and teniposide and analogues and derivatives thereof, which process comprises administering to the cell a composition as defined in any one of claims 1 to 15.

18. Use, in the manufacture of a medicament for use in the treatment of cancer, of an aqueous dispersion of micelles having an average diameter of less than 300 nm, said micelles comprising:

(a) a podophyllotoxin selected from etoposide and teniposide and analogues and derivatives thereof; and
(b) tocoferol covalently linked to a water-soluble polymer.

**Patentansprüche**

1. Zusammensetzung, umfassend eine wässrige Dispersion von Micellen mit einem durchschnittlichen Durchmesser von weniger als 300 nm, wobei die genannten Micellen umfassen:

(a) ein Podophyllotoxin ausgewählt aus Etoposid und Teniposid und Analogen und Derivaten derselben, und

(b) an ein wasserlösliches Polymer kovalent gebundenes Tocoferol.

2. Zusammensetzung nach Anspruch 1, wobei das Podophyllotoxin Etoposid ist.

3. Zusammensetzung nach Anspruch 1 oder 2, wobei das wasserlösliche Polymer aus Polyoxyethylen, Polyoxyethylen-polyoxypropylen-Copolymeren, Polyacrylamiden, Polyglycerolen, Polyvinylalkoholen, Polyvinylpyrrolidonen, Copolymeren von Polyvinylpyridin-N-Oxiden, Polyoxazolinen, Polyacroylmorpholinen oder Derivaten derselben ausgewählt ist.

4. Zusammensetzung nach einem der Ansprüche 1 bis 3, wobei das wasserlösliche Polymer ein Polypeptid oder ein Derivat desselben ist.

5. Zusammensetzung gemäß jedem der Ansprüche 1 bis 4, wobei das wasserlösliche Polymer ferner eine andere hydrophobe Gruppe als Tocoferol enthält.

6. Zusammensetzung nach einem der Ansprüche 1 bis 5, wobei das Tocoferol, das kovalent an ein wasserlösliches Polymer gebunden ist d-$\alpha$-Tocopherylpolyethylenglykol 1000 Succinat (TPGS) oder ein Derivat desselben ist.

7. Zusammensetzung nach Anspruch 6, wobei das TPGS in einer Konzentration von 0,02 Gewichtsprozent bis 20 Gewichtsprozent vorliegt.

8. Zusammensetzung nach Anspruch 6, wobei das TPGS in einer Konzentration von 0,02 Gewichtsprozent bis 10 Gewichtsprozent vorliegt.

9. Zusammensetzung nach Anspruch 6, wobei das TPGS in einer Konzentration von 4 Gewichtsprozent bis 10 Gewichtsprozent vorliegt.

10. Zusammensetzung nach einem der Ansprüche 1 bis 9, die zusätzlich ein Targeting-Molekül umfasst.

11. Zusammensetzung nach Anspruch 10, wobei das Targeting-Molekül eine Targeting-Funktionalität und eine lipophile Funktionalität umfasst.

12. Zusammensetzung nach Anspruch 10 oder 11, wobei die Targeting-funktionalität ein Hormon, Kohlenhydrat, Arzneimittel, Cytokin, Interleukin oder Peptid ist.

**13.** Zusammensetzung nach einem der Ansprüche 1 bis 12, wobei die Micellen einen durchschnittlichen Durchmesser von weniger als 100 nm aufweisen.

**14.** Zusammensetzung nach einem der Ansprüche 1 bis 12, wobei die Micellen einen durchschnittlichen Durchmesser von weniger als 50 nm aufweisen.

**15.** Zusammensetzung nach einem der Ansprüche 1 bis 12, wobei die Micellen einen durchschnittlichen Durchmesser von 3 bis 25 nm aufweisen.

**16.** Zusammensetzung wie sie gemäß den Ansprüchen 1 bis 15 definiert zur Verwendung als Medikament.

**17.** Verfahren des Zuführens an einer Zelle, ex vivo, von einem Podophyllotoxin, ausgewählt aus Etoposid und Teniposid und Analogen und Derivaten davon, wobei das Verfahren das Verabreichen einer Zusammensetzung wie sie gemäß den Ansprüchen 1 bis 15 definiert wurde, an eine Zelle umfaßt.

**18.** Verwendung einer wässrigen Dispersion von Micellen mit einem durchschnittlichen Durchmesser von 300 nm bei der Medikamentenherstellung für die Behandlung von Krebs, wobei die genannte Micellen umfassen:

(a) ein Podophyllotoxin, ausgewählt aus Etoposid und Teniposid und Analogen und Derivaten davon, und

(b) an ein wasserlösliches Polymer kovalent gebundenes Tocoferol.

## Revendications

**1.** Composition comprenant une dispersion aqueuse de micelles présentant un diamètre moyen inférieur à 300 nm, ces micelles comprenant :

(a) une podophyllotoxine choisie parmi l'étoposide et le téniposide et leurs analogues et dérivés ; et
(b) du tocophérol lié de façon covalente à un polymère soluble dans l'eau.

**2.** Composition selon la revendication 1, dans laquelle la podophyllotoxine est de l'étoposide.

**3.** Composition selon la revendication 1 ou 2, dans laquelle le polymère soluble dans l'eau est choisi parmi le polyoxyéthylène, les copolymères de polyoxyéthylène et polyoxypropylène, les polyacrylamides, les polyglycérols, les alcools polyvinyliques, les polyvinylpyrrolidones, les N-oxydes de polyvinylpyridine, les copolymères de N-oxyde de vinylpyridine et de vinylpyridine, les polyoxazolines, les polyacroylmorpholines, et leurs dérivés.

**4.** Composition selon l'une quelconque des revendications 1 à 3, dans laquelle le polymère soluble dans l'eau est un polypeptide ou un dérivé de celui-ci.

**5.** Composition selon l'une quelconque des revendications 1 à 4, dans laquelle le polymère soluble dans l'eau comprend, en outre, un groupe hydrophobe autre que le tocophérol.

**6.** Composition selon l'une quelconque des revendications 1 à 5, dans laquelle le tocophérol lié de façon covalente au polymère soluble dans l'eau est un succinate de d-$\alpha$-tocophéryl polyéthylène glycol 1000 (TPGS) ou un dérivé de celui-ci.

**7.** Composition selon la revendication 6, dans laquelle le TPGS est présent en une concentration comprise entre 0,02 % en poids et 20 % en poids.

**8.** Composition selon la revendication 6, dans laquelle le TPGS est présent en une concentration comprise entre 0,02 % en poids et 10 % en poids.

**9.** Composition selon la revendication 6, dans laquelle le TPGS est présent en une concentration comprise entre 4 % en poids et 10 % en poids.

**10.** Composition selon l'une quelconque des revendications 1 à 9, comprenant, en outre, une molécule de ciblage.

**11.** Composition selon la revendication 10, dans laquelle la molécule de ciblage comprend un fragment de ciblage et un fragment lipophile.

**12.** Composition selon la revendication 10 ou 11, dans laquelle le fragment de ciblage est une hormone, un carbohydrate, un médicament, un cytokine, une interleukine ou un peptide.

**13.** Composition selon l'une quelconque des revendications 1 à 12, dans laquelle les micelles présentent un diamètre moyen inférieur à 100 nm.

**14.** Composition selon l'une quelconque des revendications 1 à 12, dans laquelle les micelles présentent un diamètre moyen inférieur à 50 nm.

**15.** Composition selon l'une quelconque des revendications 1 à 12, dans laquelle les micelles présentent un diamètre moyen compris entre 3 nm et 25 nm.

**16.** Composition selon l'une quelconque des revendications 1 à 15, destinée à être utilisée comme médicament.

**17.** Procédé d'acheminement, dans une cellule, *ex vivo*, une podophyllotoxine choisie parmi l'étoposide et le téniposide et leurs analogues et dérivés, le procédé comprenant l'administration, à la cellule, d'une composition selon l'une quelconque des revendications 1 à 15.

**18.** Utilisation, dans la fabrication d'un médicament destiné à traiter le cancer, d'une dispersion aqueuse de micelles présentant un diamètre moyen inférieur à 300 nm, ces micelles comprenant :

(a) une podophyllotoxine choisie parmi l'étoposide et le téniposide et leurs analogues et dérivés ; et
(b) du tocophérol lié de façon covalente à un polymère soluble dans l'eau.